# EUROPEAN PATENT APPLICATION

(11) **EP 2 123 250 A1**
(43) Date of publication of application: **25.11.2009**
(21) Application number: 08711625.7
(22) Date of filing: 20.02.2008
(51) Int. Cl.: A61K 8/40, A61K 8/41, A61Q 5/04

(54) **HAIR-SHAPE CONTROLLING COMPOSITION**

(30) Priority: 20.02.2007 JP 2007040047
(71) Applicant: Shiseido Company, Limited, Chuo-ku Tokyo 104-8010 (JP); Toho Chemical Industry Co., Ltd., Tokyo 104-0044 (JP)
(72) Inventor: SHIBATA, Kazuya, Yokohama-shi Kanagawa 224-8558 (JP); KAWATA, Emiko, Yokohama-shi Kanagawa 224-8558 (JP); YAMASHITA, Takahiro, Yokohama-shi Kanagawa 224-8558 (JP); UEMURA, Masaaki, Yokohama-shi Kanagawa 224-8558 (JP); NAGANO, Tanemasa, Yokohama-shi Kanagawa 224-8558 (JP); KINOSHITA, Kouichi, Yokohama-shi Kanagawa 224-8558 (JP)
(74) Representative: Kroher, Jürgen
(86) International application number: PCT/JP2008/052819
(87) International publication number: WO 2008/102792

(57) **Abstract**

The invention provides a hair shape-controlling composition which has excellent applicability to hair, which causes less damage to permanent-waved hair or straightened hair, which can provide treated hair with an excellent texture as expected, and which does not impede a glossy appearance of treated hair. The hair shape-controlling composition including a first component containing a reducing agent and an alkaline agent, and a second component containing an oxidizing agent is provided by containing a specific hydroxy-ether-amine compound or a specific quaternary ammonium salt.

## Description

### Technical Field

The present invention relates to a composition for controlling the shape of hair (hereinafter may be referred to as a "hair shape-controlling composition"). More particularly, the present invention relates to a composition adopted in hair treatment for the purpose of controlling the shape of hair, such as permanent wave treatment or hair straightening treatment.

### Background Art

Permanent wave treatment or hair straightening treatment has been performed for intentionally controlling the shape of hair for the sake of fashion or appearance.

Permanent wave treatment or hair straightening treatment is carried out by applying, to hair, a first component for permanent waving or hair straightening containing a reducing agent (e.g., thioglycolic acid or a salt thereof, thiolactic acid or a salt thereof, or cysteine or a salt thereof) and an alkaline agent (e.g., aqueous ammonia, monoethanolamine, or ammonium hydrogencarbonate), thereby reduction-cleaving disulfide bonds present in proteins forming the hair and attaining a desired hair shape, and then by treating the hair with a second component containing, as a main component, an oxidizing agent (e.g., sodium bromate or hydrogen peroxide), thereby re-forming disulfide bonds at positions different from the original positions.

Generally, when hair undergoes permanent wave treatment or hair straightening treatment, the thus-treated hair tends to be damaged (i.e., the hair loses smoothness, fingers do not smoothly run through the hair, the hair becomes rough, or the hair loses moisture), and the hair shows a poor texture. In addition, since the surface of the treated hair is roughened, the hair loses a glossy appearance; i.e., the hair has an appearance markedly different from that of healthy hair, and thus the treated hair tends to make a strong impression of being apparently damaged.

Hitherto, various means have been proposed for solving such problems. For example, there has been proposed a first component for hair straightening containing gluconic acid and trehalose, which component causes less damage to straightened hair and can provide the straightened hair with moisture and softness (Patent Document 1). Also, there has been proposed a second component for permanent waving containing hydrogen peroxide, a pH buffering agent, and lanolin or a derivative thereof, in which the amounts of hydrogen peroxide and the pH buffering agent are specified, the component causing less damage to hair and exhibiting high waving performance (Patent Document 2). Also, there has been proposed a first component containing at least one species selected from the group consisting of glutamic acid and a glutamic acid derivative, a cysteine compound serving as a reducing agent, and diammonium dithiodiglycolate, which component keeps hair soft and provides treated hair with a good texture (Patent Document 3). Also, there has been proposed a hair shape-controlling composition containing glycine betaine, thioglycolic acid or a salt thereof, and dithiodiglycolic acid or a salt thereof, in which the ratio of dithiodiglycolic component to thioglyoclic component is optimized, which composition causes almost no damage to hair and exhibits a desired curling effect or a high hair-straightening effect (Patent Document 4). Also, there has been proposed a first component for hair shape control containing a guanidine derivative or an acid addition salt thereof, and a specific nonionic surfactant, which agent causes no damage to straightened hair and does not provide the hair with dryness (Patent Document 5).
Patent Document 1: Japanese Patent Application Laid-Open (*kokai*) No. 2004-26770
Patent Document 2: Japanese Patent Application Laid-Open (*kokai*) No. 2005-145870
Patent Document 3: Japanese Patent Application Laid-Open (*kokai*) No. 2005-145915
Patent Document 4: Japanese Patent Application Laid-Open (*kokai*) No. 2005-343831
Patent Document 5: Japanese Patent Application Laid-Open (*kokai*) No. 2006-16391
Patent Document 6: Japanese Patent Application Laid-Open (*kokai*) No. 2004-323495
Patent Document 7: Japanese Patent Application Laid-Open (*kokai*) No. 2004-323496

### Disclosure of the Invention

### Problems to be Solved by the Invention

However, when hair is treated with any of the means described in Patent Documents 1 to 5 so as to attain a desired waving effect or hair-straightening effect, the thus-treated hair fails to satisfactorily maintain good texture. In addition, the thus-treated hair tends to lose a glossy appearance. In contrast, when the amount of a reducing agent or an alkaline agent contained in a permanent waving (or hair-straightening) composition is regulated so that hair treated with the composition exhibits good texture and does not lose a glossy appearance, the composition exhibits insufficient waving or hair-straightening effect. Thus, there has not yet been provided a hair shape-controlling composition which exhibits a satisfactory hair shape-controlling effect and a satisfactory hair damage-preventing effect.

An object of the present invention is to provide a hair shape-controlling composition (specifically, a permanent wave composition or a hair straightening composition) which has excellent applicability to hair, which causes less damage to permanent-waved hair or straightened hair, which can provide treated hair with an excellent texture as expected, and which does not impede a glossy appearance of treated hair. Means for Solving the Problems

In view of the foregoing, the present inventors have conducted extensive studies, and as a result have found that the aforementioned problems can be solved by incorporating a specific surfactant into a two-component hair shape-controlling composition (i.e., a two-component permanent wave composition or a two-component hair straightening composition). The present invention has been accomplished on the basis of this finding.

Accordingly, the present invention provides a hair shape-controlling composition comprising a first component containing a reducing agent and an alkaline agent, and a second component containing an oxidizing agent, **characterized in that** the composition comprises a hydroxy-ether-amine compound represented by the following formula (I) or a quaternary ammonium salt thereof represented by the following formula (II):

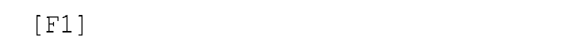

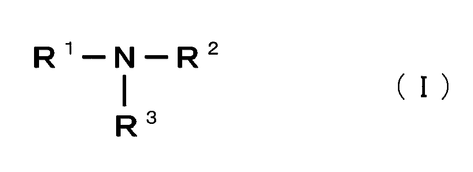

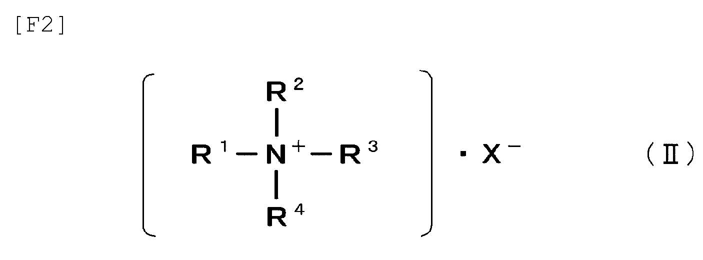

[in formulas (I) and (II), each of R¹, R², R³, and R⁴ represents a C1 to C3 alkyl group, a C1 to C3 hydroxyalkyl group, or a group represented by the following formula (III); at least one of R¹ to R³ is a group represented by the following formula (III); and X represents a halogen atom or a C1 or C2 organic sulfur acid group]:

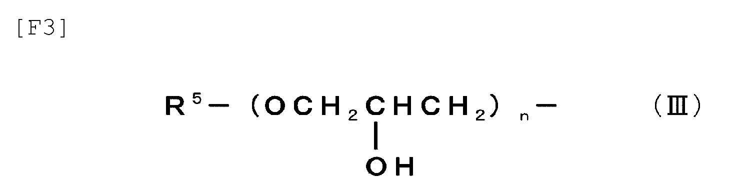

[in formula (III), R⁵ represents a linear or branched C6 to C24 alkyl, alkenyl, or hydroxyalkyl group; and n is an integer from 1 to 5] (hereinafter the hair shape-controlling composition may be referred to as "the present composition").

As used herein, "hair shape-controlling composition" encompasses a "permanent waving composition" adopted for waving hair, and a "hair-straightening composition" adopted for straightening curly hair. According to the standard of the Japanese Health, Labor, and Welfare Ministry, these two types of compositions are collectively called "permanent waving agents." However, in the present specification, the expression "hair shape-controlling composition" is adopted, in order to clearly distinguish between these compositions. "Permanent waving composition" and "hair-straightening composition" have a point of commonality in that they involve a process in which disulfide bonds in hair are cleaved by a first component, and new disulfide bonds are formed by a second component.

### Effects of the Invention

According to the present invention, there is provided a hair shape-controlling composition which can be adopted as a permanent waving composition or a hair-straightening composition, and which shows the following characteristics:
(1) excellent applicability to hair, (2) causing less damage to treated hair, (3) provision of treated hair with a good texture, and (4) maintenance of a glossy appearance of treated hair. When the present composition is adopted as a hair-straightening composition (permanent waving composition for straightening hair), curly hair is particularly effectively straightened, and the entirety of the treated hair can be provided with volume.

### Brief Description of the Drawings

[Fig. 1]

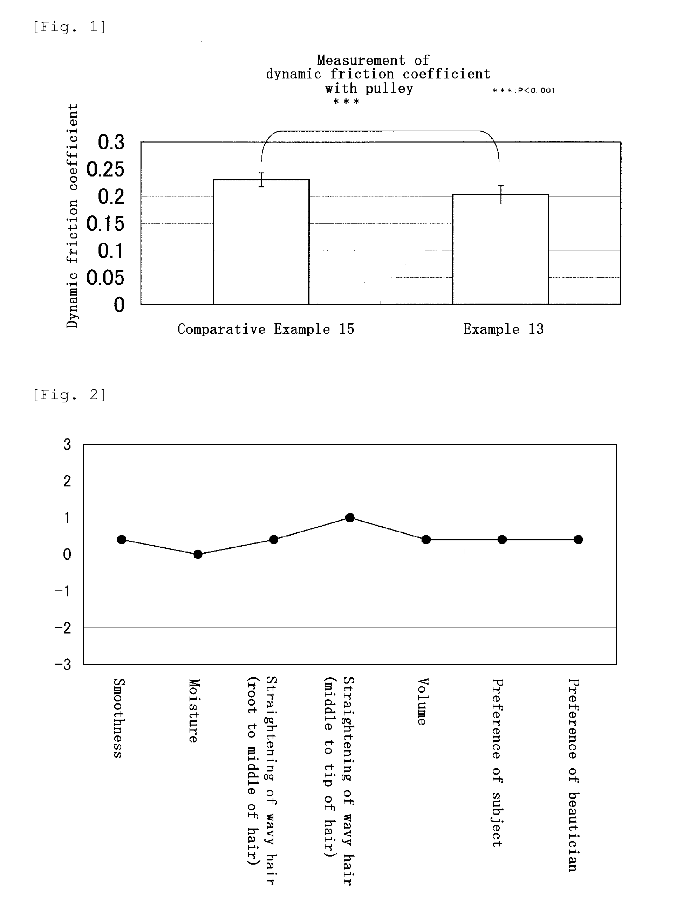

   Fig. 1 shows comparison of data on dynamic friction coefficient of hair treated with test samples, the dynamic friction coefficient quantitatively representing smoothness of the treated hair.
[Fig. 2]
   Fig. 2 shows the results of a test in which test samples were actually used by female panelists. Best Modes for Carrying Out the Invention

### [Essential component of the present composition]

As described above, the present composition contains a hydroxy-ether-amine compound represented by formula (I) (hereinafter may be referred to as a "component (I)") or a quaternary ammonium salt thereof represented by formula (II) (hereinafter may be referred to as a "component (II)"). The composition exhibits the aforementioned effects by virtue of the presence of such a component.

The component (I) or (II) may be produced through a known production method (see, for example, Patent Document 6 or 7) or may be a commercially available product (e.g., Catinal SHPA-80: N-(2-hydroxy-3-stearoxypropyl-N,N-dimethylamine, product of Toho Chemical Industry Co., Ltd.).

In the formulas representing the components (I) and (II), each of R¹ to R⁴ represents a C1 to C3 alkyl group such as methyl, ethyl, propyl, or isopropyl, or a C1 to C3 hydroxyalkyl group such as methoxy, ethoxy, propoxy, or isopropoxy.

Alternatively, each of R¹ to R⁴ represents a group represented by formula (III) wherein R⁵ represents a linear or branched C6 to C24 alkyl, alkenyl, or hydroxyalkyl group. Examples of the linear or branched C6 to C24 alkyl group include, but are not limited to, hexyl, heptyl, octyl, nonyl, decyl, undecyl, lauryl, tridecyl, myristyl, pentadecyl, palmityl, heptadecyl, stearyl, nonadecyl, icosyl, behenyl, tricosyl, and tetracosyl. Examples of the linear or branched C6 to C24 alkenyl group include, but are not limited to, myristoyl, palmitoyl, oleyl, and linoleyl. Examples of the linear or branched C6 to C24 hydroxyalkyl group include, but are not limited to, hexyloxy, heptyloxy, octyloxy, nonyloxy, decyloxy, undecyloxy, lauryloxy, tridecyloxy, myristyloxy, pentadecyloxy, palmityloxy, heptadecyloxy, stearyloxy, nonadecyloxy, icosyloxy, behenyloxy, tricosyloxy, and tetracosyloxy.

X of the quaternary ammonium salt (II) may be a halogen atom such as fluorine, chlorine, bromine, or iodine. Alternatively, X of the quaternary ammonium salt (II) may be a C1 or C2 organic sulfur acid group such as a sulfomethyl group or a sulfoethyl group.

In the present composition, optionally, one or more species of the hydroxy-ether-amine compound (I) or the quaternary ammonium salt (II) may be incorporated into either or both of the first component and the second component.

In the present composition, the amount of the hydroxy-ether-amine compound (I) or the quaternary ammonium salt (II) is preferably about 0.01 to about 10.0 mass%, particularly preferably 0.1 to 3.0 mass%, on the basis of the total mass of the first component and the second component of the composition.

When the amount of the hydroxy-ether-amine compound (I) or the quaternary ammonium salt (II) is less than 0.01 mass% on the basis of the total mass of the first component and the second component of the present composition, hair treated with the composition tends to lack smooth texture upon rinsing or after drying and to exhibit poor gloss, whereas when the amount of the hydroxy-ether-amine compound (I) or the quaternary ammonium salt (II) exceeds 10.0 mass% on the basis of the total mass of these components, the composition itself poses a problem in terms of stability, and tends to lack spreadability upon application thereof to hair.

### [Optional component of the present composition]

As described above, the present composition is a "two-component" hair composition containing the first component for cleaving disulfide bonds in hair, and the second component for re-forming disulfide bonds.

In addition to the hydroxy-ether-amine compound (I) or the quaternary ammonium salt (II), the present composition further contains other additives. The first component contains a reducing agent and an alkaline agent which may be generally incorporated into a first component of a two-component hair shape-controlling composition (permanent waving composition or hair-straightening composition). Examples of the reducing agent include thioglycolic acid or a salt thereof, thiolactic acid or a salt thereof, cysteine or a salt thereof, acetylcysteine or a salt thereof, cysteamine or a salt thereof, and cyclic mercapto compounds. Particularly preferably, thiolactic acid or a salt thereof is adopted as a reducing agent, since it exhibits an excellent effect of straightening curly hair during hair straightening. Examples of the alkaline agent include ammonia, alkanolamines such as monoethanolamine, inorganic ammonium salts such as ammonium hydrogencarbonate, organic ammonium salts, organic amines, inorganic alkaline agents, and basic amino acids. No particular limitation is imposed on the amounts of the reducing agent and alkaline agent contained in the first component, and the amounts may be appropriately determined in consideration of, for example, the specific intended use of the present composition, or the types of the reducing agent and alkaline agent adopted.

The second component contains an oxidizing agent which may be generally incorporated into a second component of a two-component hair shape-controlling composition (permanent waving composition or hair-straightening composition). Examples of the oxidizing agent include compounds containing a peroxide such as hydrogen peroxide, sodium peroxocarbonate, or urea peroxide; alkali metal peracid salts such as sodium perborate, sodium persulfate, and potassium monopersulfate; and products containing an oxidizing agent such as an alkali metal bromate (e.g., sodium bromate or potassium bromate). No particular limitation is imposed on the amount of the oxidizing agent contained in the second component, and the amount may be appropriately determined in consideration of, for example, the specific intended use of the present composition, or the type of the oxidizing agent adopted.

The present composition may optionally contain one or more gemini-type cationic activators represented by the following formula (IV):

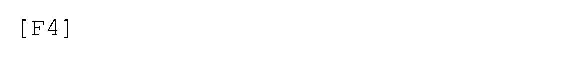

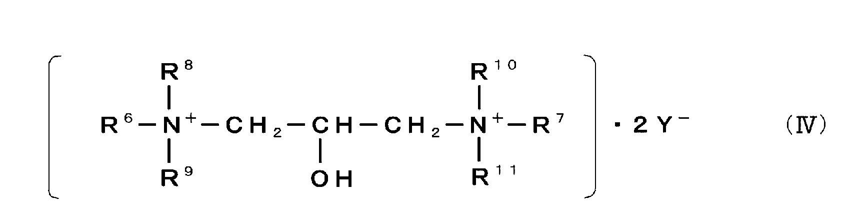

[in formula (IV), each of R⁶ and R⁷ represents a linear or branched, saturated or unsaturated C5 to C25 alkyl group; each of R⁸, R⁹, R¹⁰, and R¹¹ represents a C1 to C5 alkyl group; and Y represents a halogen atom or a C1 or C2 organic sulfur acid group]. No particular limitation is imposed on the linear or branched, saturated or unsaturated C5 to C25 alkyl group represented by R⁶ or R⁷ in formula (IV). Examples of the C5 to C25 alkyl group include the alkyl groups and alkenyl groups exemplified above for R¹ to R⁴ (C6 to C24 alkyl and alkenyl groups). Accordingly, the number of carbon atoms of the group represented by R⁶ or R⁷ falls within a range of 5 to 25. Examples of the C5 alkyl group include, but are not limited to, a pentyl group. Examples of the C25 alkyl group include, but are not limited to, a pentacosyl group. Particularly preferred is a stearyl group or a behenyl group. Each of R⁸, R⁹, R¹⁰, and R¹¹, which represents a C1 to C5 alkyl group, is particularly preferably a methyl group, an ethyl group, or a propyl group (i.e., a C1 to C3 alkyl group). Examples of the halogen atom represented by Y include fluorine, chlorine, bromine, and iodine. Examples of the C1 or C2 organic sulfur acid group include a sulfomethyl group and a sulfoethyl group.

The gemini-type cationic activator(s) (IV) may be produced through a production method disclosed in US Patent No. 4734277, 4764306, or 4812263.

In the present composition, the total amount of the gemini-type cationic activator(s) (IV) and the hydroxy-ether-amine compound (I) or the quaternary ammonium salt (II) is preferably about 0.01 to about 10.0 mass%, particularly preferably 0.1 to 3.0 mass%, on the basis of the total mass of the first component and the second component of the composition.

The present composition (first component and second component) may optionally contain, in addition to the aforementioned ingredients, an appropriate amount of an ingredient which may be generally adopted in a permanent waving composition or product or a hair-straightening composition or product.

Examples of the optionally incorporated ingredient include a humectant (e.g., propylene glycol, dipropylene glycol, or glycerin); a water-soluble polymer compound (e.g., methylcellulose or hydroxyethylcellulose); a cationic polymer compound (e.g., cationic cellulose or a copolymer of a methacryloxyethyltrimethylammonium salt and polyvinyl pyrrolidone); water-soluble silicone; a nonionic surfactant (e.g., polyoxyethylene hardened castor oil or polyoxyethylene alkyl ether); an amphoteric surfactant (e.g., 2-alkyl-N-carboxymethyl-N-hydroxyethylimidazolinium betaine or trialkylaminoacetic acid betaine); a penetrant (e.g., urea, alkyl urea, benzyl alcohol, or monoethylene oxide benzyl ether); a natural animal (or plant) extract; an amino acid; an organic acid (e.g., citric acid or lactic acid); an iorganic salt (e.g., sodium chloride or potassium chloride); a perfume; a preservative (e.g., paraben); a metal ion sequestering agent (e.g., EDTA-3Na); a UV absorbent (e.g., oxybenzone); and a dye.

No particular limitation is imposed on the form of the present composition (first component or second component), and the composition may be provided in the form of non-emulsified product or emulsified product (oil-in-water type or water-in-oil type). The present composition in the form of, for example, liquid, emulsion, or cream may be produced through a customary method.

### Examples

The present invention will next be described in mode detail by way of examples, which should not be construed as limiting the invention thereto. Unless otherwise specified, the amount of a component incorporated is represented by "mass%."

### [Test Example 1] Texture test using hair bundle

### <Test method>

For evaluation of the effects of the present invention, in the present Example, hair bundles were subjected to a test adopting permanent wave treatment and hair straightening treatment.

### (1) Permanent wave treatment

A plurality of hair bundles (each having a weight of 0.7 g and a length of 20 cm) were prepared from non-chemically damaged hair fibers collected from a single person. The thus-prepared hair bundles were immersed in a commercially available breaching product (ammonia: 0.8%, hydrogen peroxide: 3.0%, potassium persulfate: 0.1%) for 20 minutes so that the hair bundles were damaged to the same extent. Each of the thus-bleached hair bundles was wound around a plastic rod having a diameter of 1.5 cm, and a first component (2.0 mL) was applied to the hair bundle. The hair bundle was allowed to stand at 25°C for 15 minutes and then washed with water. Subsequently, a second component (2.0 mL) was applied to the hair bundle, and the hair bundle was allowed to stand at 25°C for 15 minutes and then washed with water, followed by natural drying.

### (2) Hair straightening treatment

In a manner similar to the case of the permanent wave composition described above in (1), bleached hair bundles were prepared. The first component (2.0 g) was applied to each of the bleached hair bundles, and the hair bundle was allowed to stand at 25°C for 15 minutes and then washed with water, followed by drying with a towel. Thereafter, the thus-dried hair bundle was treated with a high-temperature styling iron set at 180°C. Subsequently, the second component (2.0 g) was applied to the hair bundle, and the hair bundle was allowed to stand at 25°C for five minutes and then washed with water, followed by natural drying.

### (3) Evaluation

### (a) Applicability (easy application to hair)

Applicability of the test sample (first component or second component) to a bleached hair bundle (i.e., easy application to hair) was organoleptically evaluated by 12 expert panelists according to the following criteria:
AA: 10 or more of the 12 expert panelists answered that the test sample was easy to apply to hair;
BB: 7 to 9 of the 12 expert panelists answered that the test sample was easy to apply to hair;
CC: 4 to 6 of the 12 expert panelists answered that the test sample was easy to apply to hair; and
DD: 3 or less of the 12 expert panelists answered that the test sample was easy to apply to hair.

### (b) Damage

The degree of damage of a hair bundle after the final natural drying in the aforementioned treatment (1) or (2) was organoleptically evaluated by 12 expert panelists according to the following criteria:
AA: 10 or more of the 12 expert panelists answered that no damage was observed in the hair bundle;
BB: 7 to 9 of the 12 expert panelists answered that no damage was observed in the hair bundle;
CC: 4 to 6 of the 12 expert panelists answered that no damage was observed in the hair bundle; and
DD: 3 or less of the 12 expert panelists answered that no damage was observed in the hair bundle.

### (c) Smoothness

The degree of smoothness of a hair bundle after the final natural drying in the aforementioned treatment (1) or (2) was organoleptically evaluated by 12 expert panelists according to the following criteria:
AA: 10 or more of the 12 expert panelists answered that the hair bundle was smooth;
BB: 7 to 9 of the 12 expert panelists answered that the hair bundle was smooth;
CC: 4 to 6 of the 12 expert panelists answered that the hair bundle was smooth; and
DD: 3 or less of the 12 expert panelists answered that the hair bundle was smooth.

### (d) Gloss

The degree of gloss of a hair bundle after the final natural drying in the aforementioned treatment (1) or (2) was organoleptically evaluated by 12 expert panelists according to the following criteria:
AA: 10 or more of the 12 expert panelists answered that the hair bundle did not lose gloss;
BB: 7 to 9 of the 12 expert panelists answered that the hair bundle did not lose gloss;
CC: 4 to 6 of the 12 expert panelists answered that the hair bundle did not lose gloss; and
DD: 3 or less of the 12 expert panelists answered that the hair bundle did not lose gloss.

### <Results of Test Example 1>

Compositions of Examples 1 to 12 and Comparative Examples 1 to 14 were prepared through a customary method (for example, in Example 11, the first component in the form of emulsion was prepared by emulsifying an oily component with a surfactant, followed by sequential addition of an active component, a stabilizer, and the like; and the second component was prepared in a similar manner) (Table 1 shows liquid-type compositions having low viscosity, and Table 2 shows emulsion-type and cream-type compositions (in Tables 1 and 2, the amounts of all components are represented by mass%)). Each of the thus-prepared compositions was tested through the above-described test methods (procedures) (1) to (3). The results of the test are also shown in Tables 1 and 2. "Hydroxy-ether-amine compound (1)" shown in Tables 1 and 2 corresponds to a hydroxy-ether-amine compound represented by formula (I) wherein R¹ is a group represented by formula (III) wherein R⁵ is C₁₈H₃₇, n is 1, and each of R² and R³ is a methyl group; i.e., hydroxy-ether-amine compound (1) is stearoxyhydroxypropyldimethylamine. "Hydroxy-ether-amine quaternary ammonium salt (2)" shown in Tables 1 and 2 corresponds a quaternary ammonium salt of a hydroxy-ether-amine compound represented by formula (II) wherein R¹ is a group represented by formula (III) wherein R⁵ is C₁₈H₃₇, n is 1, each of R², R³, and R⁴ is a methyl group, and X is a chlorine atom. These compounds, which can be readily produced through disclosed techniques, were obtained as commercial products from Toho Chemical Industry Co., Ltd.

Hydroxy-ether-amine compounds and hydroxy-ether-amine quaternary ammonium salts other than the aforementioned hydroxy-ether-amine compound (1) and hydroxy-ether-amine quaternary ammonium salt (2) were employed in Examples 13 to 21. Specific data of these essential components (together with data of the above-defined substituents) are disclosed in formulations of Examples 13 to 21.

**[Table 1-1]**

| | | Ex. 1 | Ex. 2 | Ex. 3 | Ex. 4 | Ex. 5 | Ex. 6 |
|---|---|---|---|---|---|---|---|
| | Hydroxy-ether-amine compound (1) | 0.2 | - | - | - | 0.2 | - |
| | Hydroxy-ether-amine quaternary ammonium salt (2) | - | 0.2 | - | - | - | 0.2 |
| | Stearyltrimethylammonium chloride | - | - | - | - | - | |
| | Distearyldimethylammonium chloride | - | - | - | - | - | - |
| | Lauryltrimethylammonium chloride | - | - | - | - | - | - |
| | POE (20) cetyl ether | 0.5 | 0.5 | 0.5 | 0.5 | 0.5 | 0.5 |
| | Ammonium thioglycolate (50%) | 12.0 | 12.0 | 12.0 | 12.0 | 12.0 | 12.0 |
| | Aqueous ammonia (28%) | 1.0 | 1.0 | 1.0 | 1.0 | 1.0 | 1.0 |
| | Monoethanolamine | 1.0 | 1.0 | 1.0 | 1.0 | 1.0 | 1.0 |
| | Ammonium carbonate | 1.0 | 1.0 | 1.0 | 1.0 | 1.0 | 1.0 |
| | Dipropylene glycol | 3.0 | 3.0 | 3.0 | 3.0 | 3.0 | 3.0 |
| | Disodium edetate | 0.5 | 0.5 | 0.5 | 0.5 | 0.5 | 0.5 |
| | Perfume | Small amount | Small amount | Small amount | Small amount | Small amount | Small amount |
| | Purified water | Balance | Balance | Balance | Balance | Balance | Balance |
| | Total | 100 | 100 | 100 | 100 | 100 | 100 |
| | | | | | | | |
| | Hydroxy-ether-amine compound (1) | - | - | 0.2 | - | 0.2 | - |
| | ydroxy-ether-amine quaternary ammonium salt (2) | - | - | - | 0.2 | - | 0.2 |
| | tearyltrimethylammonium chloride | - | - | - | - | - | - |
| | Distearyldimethylammonium chloride | - | - | - | - | - | - |
| | Lauryltrimethylammonium chloride | - | - | - | - | - | - |
| | POE (30) stearyl ether | 0.5 | 0.5 | 0.5 | 0.5 | 0.5 | 0.5 |
| | Sodium bromate | 7.0 | 7.0 | 7.0 | 7.0 | 7.0 | 7.0 |
| | Cetyl alcohol | 1.0 | 1.0 | 1.0 | 1.0 | 1.0 | 1.0 |
| | pH-buffering agent | Appropriate amount | Appropriate amount | Appropriate amount | Appropriate amount | Appropriate amount | Appropriate amount |
| | Purified water | Balance | Balance | Balance | Balance | Balance | Balance |
| | Total | 100 | 100 | 100 | 100 | 100 | 100 |
| | | | | | | | |
| Evaluation | Applicability of first agent (easy application to hair) | BB | BB | BB | BB | BB | BB |
| | Applicability of second agent (easy application to hair) | CC | CC | BB | BB | BB | BB |
| | Organoleptic test (damage) | AA | AA | BB | BB | AA | AA |
| | Organoleptic test (smoothness) | AA | AA | AA | AA | AA | AA |
| | Organoleptic test (gloss) | BB | BB | AA | AA | AA | AA |

**[Table 1-2]**

| | | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|---|
| | Hydroxy-ether-amine compound (1) | - | - | - | - | - | - | - |
| | ydroxy-ether-amine quaternary ammonium salt (2) | - | - | - | - | - | - | - |
| | Stearyltrimethylammonium chloride | - | 0.2 | - | - | - | - | - |
| | Distearyldimethylammonium chloride | - | - | 0.2 | - | - | - | - |
| | Lauryltrimethylammonium chloride | - | - | - | 0.2 | - | - | - |
| | POE (20) cetyl ether | 0.5 | 0.5 | 0.5 | 0.5 | 0.5 | 0.5 | 0.5 |
| | Ammonium thioglycolate (50%) | 12.0 | 12.0 | 12.0 | 12.0 | 12.0 | 12.0 | 12.0 |
| | Aqueous ammonia (28%) | 1.0 | 1.0 | 1.0 | 1.0 | 1.0 | 1.0 | 1.0 |
| | Monoethanolamine | 1.0 | 1.0 | 1.0 | 1.0 | 1.0 | 1.0 | 1.0 |
| | Ammonium carbonate | 1.0 | 1.0 | 1.0 | 1.0 | 1.0 | 1.0 | 1.0 |
| | Dipropylene glycol | 3.0 | 3.0 | 3.0 | 3.0 | 3.0 | 3.0 | 3.0 |
| | Disodium edetate | 0.5 | 0.5 | 0.5 | 0.5 | 0.5 | 0.5 | 0.5 |
| | Perfume | Small amount | Small amount | Small amount | Small amount | Small amount | Small amount | Small amount |
| | Purified water | Balance | Balance | Balance | Balance | Balance | Balance | Balance |
| | Total | 100 | 100 | 100 | 100 | 100 | 100 | 100 |
| | | | | | | | | |
| | Hydroxy-ether-amine compound (1) | - | - | - | - | - | - | - |
| | Hydroxy-ether-amine quaternary ammonium salt (2) | - | - | - | - | - | - | - |
| | Stearyltrimethylammonium chloride | - | - | - | - | 0.2 | - | - |
| | Distearyldimethylammonium chloride | - | - | - | - | - | 0.2 | - |
| | Lauryltrimethylammonium chloride | - | - | - | - | - | - | 0.2 |
| | POE (30) stearyl ether | 0.5 | 0.5 | 0.5 | 0.5 | 0.5 | 0.5 | 0.5 |
| | Sodium bromate | 7.0 | 7.0 | 7.0 | 7.0 | 7.0 | 7.0 | 7.0 |
| | Cetyl alcohol | 1.0 | 1.0 | 1.0 | 1.0 | 1.0 | 1.0 | 1.0 |
| | pH-buffering agent | Appropriate amount | Appropriate amount | Appropriate amount | Appropriate amount | Appropriate amount | Appropriate amount | Appropriate amount |
| | Purified water | Balance | Balance | Balance | Balance | Balance | Balance | Balance |
| | Total | 100 | 100 | 100 | 100 | 100 | 100 | 100 |
| | | | | | | | | |
| Evaluation | Applicability of first agent (easy application to hair) | BB | BB | BB | BB | BB | BB | BB |
| | Applicability of second agent (easy application to hair) | CC | CC | CC | CC | CC | CC | CC |
| | Organoleptic test (damage) | CC | CC | CC | CC | CC | CC | CC |
| | Organoleptic test (smoothness) | CC | CC | CC | CC | CC | CC | CC |
| | Organoleptic test (gloss) | CC | CC | CC | CC | CC | CC | CC |

**[Table 2-1]**

| | | Ex. 7 | Ex. 8 | Ex. 9 | Ex. 10 | Ex. 11 | Ex. 12 |
|---|---|---|---|---|---|---|---|
| | Hydroxy-ether-amine compound (1) | 2.0 | - | - | - | 2.0 | - |
| | Hydroxy-ether-amine quaternary ammonium salt (2) | - | 2.0 | - | - | - | 2.0 |
| | Stearyltrimethylammonium chloride | - | - | - | - | - | - |
| | Distearyldimethylammonium chloride | - | - | - | - | - | - |
| | Lauryltrimethylammonium chloride | - | - | - | - | - | - |
| | POE (5) behenyl ether | 2.0 | 2.0 | 2.0 | 2.0 | 2.0 | 2.0 |
| | POE (20) cetyl ether | 2.0 | 2.0 | 2.0 | 2.0 | 2.0 | 2.0 |
| | Ammonium thioglycolate (50%) | 15.0 | 15.0 | 15.0 | 15.0 | 15.0 | 15.0 |
| | Diammonium dithiodiglycolate (40%) | 3.0 | 3.0 | 3.0 | 3.0 | 3.0 | 3.0 |
| | Cetyl alcohol | 5.0 | 5.0 | 5.0 | 5.0 | 5.0 | 5.0 |
| | Monoethanolamine | 2.0 | 2.0 | 2.0 | 2.0 | 2.0 | 2.0 |
| | Propylene glycol | 3.0 | 3.0 | 3.0 | 3.0 | 3.0 | 3.0 |
| | Disodium edetate | 0.5 | 0.5 | 0.5 | 0.5 | 0.5 | 0.5 |
| | Perfume | Small amount | Small amount | Small amount | Small amount | Small amount | Small amount |
| | Purified water | Balance | Balance | Balance | Balance | Balance | Balance |
| | Total | 100 | 100 | 100 | 100 | 100 | 100 |
| | | | | | | | |
| | Hydroxy-ether-amine compound (1) | - | - | 2.0 | - | 2.0 | - |
| | Hydroxy-ether-amine quaternary ammonium salt (2) | - | - | - | 2.0 | | 2.0 |
| | Stearyltrimethylammonium chloride | - | - | - | - | - | - |
| | Distearyldimethylammonium chloride | - | - | - | - | - | - |
| | Lauryltrimethylammonium chloride | - | - | - | - | - | - |
| | POE (30) stearyl ether | 2.0 | 2.0 | 2.0 | 2.0 | 2.0 | 2.0 |
| | Hydrogen peroxide (35%) | 6.0 | 6.0 | 6.0 | 6.0 | 6.0 | 6.0 |
| | Behenyl alcohol | 3.0 | 3.0 | 3.0 | 3.0 | 3.0 | 3.0 |
| | pH-buffering agent | Appropriate amount | Appropriate amount | Appropriate amount | Appropriate amount | Appropriate amount | Appropriate amount |
| | Purified water | Balance | Balance | Balance | Balance | Balance | Balance |
| | Total | 100 | 100 | 100 | 100 | 100 | 100 |
| | | | | | | | |
| Evaluation | Applicability of first agent (easy application to hair) | BB | BB | DD | DD | BB | BB |
| | Applicability of second agent (easy application to hair) | DD | DD | BB | BB | BB | BB |
| | Organoleptic test (damage) | BB | BB | CC | CC | AA | AA |
| | Organoleptic test (smoothness) | AA | AA | AA | AA | AA | AA |
| | Organoleptic test (gloss) | BB | BB | AA | AA | AA | AA |

**[Table 2-2]**

| | | Comp. Ex. 8 | Comp. Ex. 9 | Comp. Ex. 10 | Comp. Ex. 11 | Comp. Ex. 12 | Comp. Ex. 13 | Comp. Ex. 14 |
|---|---|---|---|---|---|---|---|---|
| | Hydroxy-ether-amine compound (1) | - | - | - | - | - | - | - |
| | Hydroxy-ether-amine quaternary ammonium salt (2) | - | - | - | - | - | - | - |
| | Stearyltrimethylammonium chloride | - | 2.0 | - | - | - | - | - |
| | Distearyldimethylammonium chloride | - | - | 2.0 | - | - | - | - |
| | Lauryltrimethylammonium chloride | - | - | - | 2.0 | - | - | - |
| | POE (5) behenyl ether | 2.0 | 2.0 | 2.0 | 2.0 | 2.0 | 2.0 | 2.0 |
| | POE (20) cetyl ether | 2.0 | 2.0 | 2.0 | 2.0 | 2.0 | 2.0 | 2.0 |
| | Ammonium thioglycolate (50%) | 15.0 | 15.0 | 15.0 | 15.0 | 15.0 | 15.0 | 15.0 |
| | Diammonium dithiodiglycolate (40%) | 3.0 | 3.0 | 3.0 | 3.0 | 3.0 | 3.0 | 3.0 |
| | Cetyl alcohol | 5.0 | 5.0 | 5.0 | 5.0 | 5.0 | 5.0 | 5.0 |
| | Monoethanolamine | 2.0 | 2.0 | 2.0 | 2.0 | 2.0 | 2.0 | 2.0 |
| | Propylene glycol | 3.0 | 3.0 | 3.0 | 3.0 | 3.0 | 3.0 | 3.0 |
| | Disodium edetate | 0.5 | 0.5 | 0.5 | 0.5 | 0.5 | 0.5 | 0.5 |
| | Perfume | Small amount | Small amount | Small amount | Small amount | Small amount | Small amount | Small amount |
| | Purified water | Balance | Balance | Balance | Balance | Balance | Balance | Balance |
| | Total | 100 | 100 | 100 | 100 | 100 | 100 | 100 |
| | | | | | | | | |
| | Hydroxy-ether-amine compound (1) | - | - | - | - | - | - | - |
| | Hydroxy-ether-amine quaternary ammonium salt (2) | - | - | - | - | - | - | - |
| | Stearyltrimethylammonium chloride | - | - | - | - | 2.0 | - | - |
| | Distearyldimethylammonium chloride | - | - | - | - | - | 2.0 | - |
| | Lauryltrimethylammonium chloride | - | - | - | - | - | - | 2.0 |
| | POE (30) stearyl ether | 2.0 | 2.0 | 2.0 | 2.0 | 2.0 | 2.0 | 2.0 |
| | Hydrogen peroxide (35%) | 6.0 | 6.0 | 6.0 | 6.0 | 6.0 | 6.0 | 6.0 |
| | Behenyl alcohol | 3.0 | 3.0 | 3.0 | 3.0 | 3.0 | 3.0 | 3.0 |
| | pH-buffering agent | Appropriate amount | Appro-priate amount | Appropriate amount | Appro-priate amount | Appropriate amount | Appropriate amount | Appropriate amount |
| | Purified water | Balance | Balance | Balance | Balance | Balance | Balance | Balance |
| | Total | 100 | 100 | 100 | 100 | 100 | 100 | 100 |
| | | | | | | | | |
| Evaluation | Applicability of first agent (easy application to hair) | DD | DD | DD | DD | DD | DD | DD |
| | Applicability of second agent (easy application to hair) | DD | DD | DD | DD | DD | DD | DD |
| | Organoleptic test (damage) | DD | DD | DD | DD | DD | DD | DD |
| | Organoleptic test (smoothness) | CC | CC | CC | CC | CC | CC | CC |
| | Organoleptic test (gloss) | CC | CC | CC | CC | CC | CC | CC |

Data shown in Tables 1 and 2 indicate the following. In the case of liquid-type compositions of low viscosity, no considerable difference is observed in applicability between Examples and Comparative Examples (comparison between Examples 1 and 2 and Comparative Examples 1 to 4), whereas in the case of emulsion-type or cream-type compositions, applicability is clearly higher in Examples than in Comparative Examples (comparison between Examples 7 and 8 and Comparative Examples 8 to 11). As shown in Tables 1 and 2, compositions of the Examples cause very little damage to treated hair, provide treated hair with smooth texture, and reduce loss of a glossy appearance of treated hair. In contrast, compositions of the Comparative Examples are inferior to those of the Examples in terms of applicability, damage to hair, smoothness, and gloss; i.e., compositions of the Comparative Examples fail to attain the object of the present invention.

As is also clear from these data, when the hydroxy-ether-amine compound (I) or the hydroxy-ether-amine quaternary ammonium salt (II) is incorporated only into the first component, the resultant composition very effectively serves to provide hair with smoothness and gloss; when the compound (I) or the ammonium salt (II) is incorporated only into the second component, the resultant composition very effectively serves to reduce damage to hair and to provide hair with smoothness; and when the compound (I) or the ammonium salt (II) is incorporated into both the first component and the second component, the resultant composition very effectively serves to reduce damage to hair and to provide hair with smoothness and gloss (for example, from comparison between Examples 1 and 2, Examples 3 and 4, and Examples 5 and 6). The above-described data strongly suggest that when the composition is in the form of emulsion or cream, incorporation of the hydroxy-ether-amine compound (I) or the hydroxy-ether-amine quaternary ammonium salt (II) into both the first component and the second component is important for providing the final products of the composition with good applicability (for example, from comparison between Examples 7 and 8 and Examples 9 and 10).

### [Test Example 2] Measurement of dynamic friction coefficient in hair bundle

The aforementioned "smooth texture" was further examined through measurement of the dynamic friction coefficient of the surface of hair.

In a manner similar to the case of the permanent wave treatment (1) or the hair straightening treatment (2) described above in Test Example 1, bleached hair bundles were prepared. The first component of Example 13 (2.0 g), which had been prepared according to the below-described formulation through a customary method, was applied to each of the bleached hair bundles, and the hair bundle was allowed to stand at 25°C for 20 minutes and then washed with water, followed by drying with a towel. Thereafter, the thus-dried hair bundle was treated with a high-temperature styling iron set at 180°C. Subsequently, the second component of Example 13 (2.0 g) was applied to the hair bundle, and the hair bundle was allowed to stand at 25°C for five minutes and then washed with water, followed by drying with a towel and natural drying. Six hair fibers were randomly selected from the thus-dried hair bundle, and the dynamic friction coefficient of the surface of the hair fibers was measured through a customary method by means of a pulley tester. In this dynamic friction coefficient test, the composition of Comparative Example 15, which had been prepared by removing a hydroxy-ether-amine compound (1) from the composition of Example 13, was adopted as a control composition. The test results are shown in Fig. 1 (wherein the vertical axis represents dynamic friction coefficient). The dynamic friction coefficient of the surface of hair fibers treated with the composition of Example 13 was found to be significantly lower than that of the surface of hair fibers treated with the composition of Comparative Example 15; i.e., these quantitative data showed that hair treated with the composition of Example 13 was smoother than hair treated with the composition of Comparative Example 15.

### [Example 13] (1st component: cream, 2nd component: emulsion)

| [First component] | Formulation |
|---|---|
| | (mass%) |
| Ammonium thiolactate solution (60%) | 16.0 |
| Liquid paraffin | 2.0 |
| Cetyl alcohol | 7.0 |
| Propylene glycol | 4.0 |
| Polyoxyethylene oleyl ether | 1.0 |
| Alkyltrimethylammonium chloride solution | 1.5 |
| Hydroxy-ether-amine compound (R¹= formula (III)/R⁵=C₁₈H₃₇, R²⁻³=CH_{3,} n=1) | 0.2 |
| Monoethanolamine solution | 3.0 |
| Diammonium dithiodiglycolate solution | 5.0 |
| Perfume | 0.2 |
| Purified water | balance |
| Total | 100.0 |
| | |

| [Second component] | Formulation (mass%) |
|---|---|
| Aqueous hydrogen peroxide (30.0%) | 6.0 |
| Liquid paraffin | 1.0 |
| Cetyl alcohol | 2.0 |
| Polyoxyethylene cetyl ether | 0.5 |
| Polyoxyethylene oleyl ether | 0.1 |
| Alkyltrimethylammonium chloride solution | 0.5 |
| Phosphoric acid | 0.1 |
| Sodium monohydrogenphosphate | 0.2 |
| Perfume | 0.1 |
| Purified water | balance |
| Total | 100.0 |

### [Comp. Ex. 15] (1st component: cream, 2nd component: emulsion)

| [First component] | Formulation |
|---|---|
| | (mass%) |
| Ammonium thiolactate solution (60%) | 16.0 |
| Liquid paraffin | 2.0 |
| Cetyl alcohol | 7.0 |
| Propylene glycol | 4.0 |
| Polyoxyethylene oleyl ether | 1.0 |
| Alkyltrimethylammonium chloride solution | 1.5 |
| Monoethanolamine solution | 3.0 |
| Diammonium dithiodiglycolate solution | 5.0 |
| Perfume | 0.2 |
| Purified water | balance |
| Total | 100.0 |
| | |

| [Second component] | Formulation (mass%) |
|---|---|
| Aqueous hydrogen peroxide (30.0%) | 6.0 |
| Liquid paraffin | 1.0 |
| Cetyl alcohol | 2.0 |
| Polyoxyethylene cetyl ether | 0.5 |
| Polyoxyethylene oleyl ether | 0.1 |
| Alkyltrimethylammonium chloride solution | 0.5 |
| Phosphoric acid | 0.1 |
| Sodium monohydrogenphosphate | 0.2 |
| Perfume | 0.1 |
| Purified water | balance |
| Total | 100.0 |

### [Test Example 3] Half-head comparative test

For more practical examination of the present composition, five female panelists having naturally wavy hair of about 30 cm to 50 cm or longer were subjected to a half-head comparative test carried out by five beauticians adopting the composition. The practical examination was carried out by using, as test samples, the composition prepared according to the below-described formulation of Example 14 through a customary method (i.e., the present composition), and the composition of Comparative Example 16, which had been prepared by removing a hydroxy-ether-amine compound (1) from the composition of Example 14.

The five female panelists having naturally wavy hair were respectively tested by five different beauticians. Specifically, each beautician applied the test samples of Example 14 and Comparative Example 16 to hair of the corresponding female panelist on the right half side and left half side of the head, respectively. Firstly, the first component was applied to the hair, and then the hair was allowed to stand for 20 minutes. The first component was rinsed off, and then the hair was completely dried with a dryer. Subsequently, the hair was treated with a high-temperature styling iron set at 180°C, and the second component was applied to the hair. Thereafter, the hair was allowed to stand for three minutes, and then the second component was rinsed off. Thus, the entire process was completed. After completion of the process, the five female panelists (subjects) and the five beautician panelists evaluated the present composition in terms of (a) smoothness of hair, (b) moisture, (c) straightening of wavy hair (the root to the middle of hair), (d) straightening of wavy hair (the middle to the tip of hair), (e) volume of hair, (f) preference of the subjects, and (g) preference of the beauticians. Specifically, the present composition was evaluated on these items according to the following ratings (based on the difference from the composition of Comparative Example 16 (comparative composition)):
score 3: much better than the comparative composition;
score 2: considerably better than the comparative composition;
score 1: better than the comparative composition;
score 0: no difference between the present composition and the comparative composition;
score -1: worse than the comparative composition;
score -2: considerably worse than the comparative composition; and
score -3: much worse than the comparative composition. Scores of each of the aforementioned items evaluated by the 10 panelists were averaged (note: the average score of preference of the subjects (or beauticians) was based on evaluation by the corresponding five panelists). The results are shown in Fig. 2 (wherein the vertical axis represents average score).

Data obtained in Test Example 3 indicate that the present composition is excellent in terms of smoothness of hair, volume of hair, and preference, particularly in terms of "straightening of wavy hair."

### [Example 14] (1st component: cream, 2nd component: emulsion)

| [First component] | Formulation |
|---|---|
| | (mass%) |
| Ammonium thiolactate solution (60%) | 17.0 |
| Liquid paraffin | 1.0 |
| Cetyl alcohol | 5.0 |
| Propylene glycol | 2.0 |
| Polyoxyethylene oleyl ether | 0.5 |
| Alkyltrimethylammonium chloride solution | 0.5 |
| Hydroxy-ether-amine compound | 0.5 |
| (R¹= formula (III)/R⁵=C₁₈H₃₇, R²⁻³=CH₃, n=1) | |
| Monoethanolamine solution | 2.0 |
| Diammonium dithiodiglycolate solution | 7.0 |
| Perfume | 0.4 |
| Purified water | balance |
| Total | 100.0 |
| | |

| [Second component] | Formulation |
|---|---|
| | (mass%) |
| Aqueous hydrogen peroxide (30.0%) | 4.0 |
| Liquid paraffin | 0.2 |
| Cetyl alcohol | 0.5 |
| Polyoxyethylene cetyl ether | 0.1 |
| Alkyltrimethylammonium chloride solution | 0.2 |
| Phosphoric acid | 0.5 |
| Sodium monohydrogenphosphate | 0.5 |
| Perfume | 0.1 |
| Purified water | balance |
| Total | 100.0 |

### [Comp. Ex. 16] (1st component: cream, 2nd component: emulsion)

| [First component] | Formulation |
|---|---|
| | (mass%) |
| Ammonium thiolactate solution (60%) | 17.0 |
| Liquid paraffin | 1.0 |
| Cetyl alcohol | 5.0 |
| Propylene glycol | 2.0 |
| Polyoxyethylene oleyl ether | 0.5 |
| Alkyltrimethylammonium chloride solution | 0.5 |
| Monoethanolamine solution | 2.0 |
| Diammonium dithiodiglycolate solution | 7.0 |
| Perfume | 0.4 |
| Purified water | balance |
| Total | 100.0 |
| | |

| [Second component] | Formulation |
|---|---|
| | (mass%) |
| Aqueous hydrogen peroxide (30.0%) | 4.0 |
| Liquid paraffin | 0.2 |
| Cetyl alcohol | 0.5 |
| Polyoxyethylene cetyl ether | 0.1 |
| Alkyltrimethylammonium chloride solution | 0.2 |
| Phosphoric acid | 0.5 |
| Sodium monohydrogenphosphate | 0.5 |
| Perfume | 0.1 |
| Purified water | balance |
| Total | 100.0 |

Formulation examples of the present composition will be described below (Examples 15 to 23). In each formulation example, a composition was prepared through a customary method corresponding to its form. Emulsion-type or cream-type compositions exhibit excellent applicability, cause very little damage to treated hair, provide treated hair with smooth texture, and reduce loss of a glossy appearance of treated hair. Liquid-type compositions exhibit applicability comparable to or higher than that of conventional ones, cause very little damage to treated hair, provide treated hair with smooth texture, and reduce loss of a glossy appearance of treated hair. When each of the compositions of these Examples is adopted as a hair-straightening composition (permanent waving composition for straightening hair), curly hair is effectively straightened, and the entirety of the treated hair can be provided with volume.

### [Example 15] (1st component: liquid, 2nd component: liquid)

| [First component] | Formulation |
|---|---|
| | (mass%) |
| Ammonium thioglycolate solution (50%) | 16.0 |
| Diammonium dithioglycolate solution (40%) | 3.8 |
| 28% Aqueous ammonia | 1.5 |
| Monoethanolamine | 1.0 |
| Ammonium hydrogencarbonate | 1.5 |
| Hydroxy-ether-amine compound | 0.01 |
| (R¹= formula (III)/R⁵=C₁₆H₃₃, R²⁻³=CH₃, n=1) | |
| Polyoxypropylene sorbitol | 0.5 |
| Polydimethylsiloxane | 1.0 |
| Cationized hydrolyzed keratin | 0.2 |
| Disodium edetate | 0.1 |
| POE (20) lauryl ether | 0.3 |
| Perfume | 0.1 |
| Purified water | balance |
| Total | 100.0 |
| | |

| [Second component] | Formulation |
|---|---|
| | (mass%) |
| Sodium bromate | 7.0 |
| Potassium dihydrogen phosphate | 0.5 |
| Caustic soda | 0.2 |
| Sodium lauryl sulfate solution (40%) | 1.5 |
| Carboxyvinyl polymer | 0.3 |
| Polyoxyethylene sorbitol | 0.5 |
| POE (20) oleyl ether | 0.5 |
| Purified water | balance |
| Total | 100.0 |

### [Example 16] (1st component: liquid, 2nd component: liquid)

| [First component] | Formulation |
|---|---|
| | (mass%) |
| Ammonium thiolactate solution (60%) | 10.0 |
| Monoethanolamine thiolactate solution (40%) | 10.0 |
| Diammonium dithiodilactate solution (40%) | 2.5 |
| L-Arginine | 1.0 |
| Monoethanolamine | 2.0 |
| Hydroxy-ether-amine quaternary ammonium salt | 10.0 |
| (R¹= formula (III)/R⁵=C₁₈H₃₇, R²⁻⁴=CH₃, n=1, X=C₂H₅SO₄) | |
| Amino-modified silicone | 1.0 |
| Oat extract | 0.2 |
| Trisodium edetate | 0.1 |
| POE (15) cetyl ether | 0.3 |
| Perfume | 0.2 |
| Purified water | balance |
| Total | 100.0 |
| | |

| [Second component] | Formulation |
|---|---|
| | (mass%) |
| Sodium bromate | 8.0 |
| Hydroxy-ether-amine compound | 0.5 |
| (R¹= formula(III)/R⁵=C₂₂H₄₅, R²⁻³=CH_{3,} n=1) | |
| Potassium dihydrogen phosphate | 0.1 |
| Disodium hydrogen phosphate | 0.2 |
| Palm oil fatty acid methyl tauride sodium | 1.5 |
| solution (30%) | |
| Purified water | balance |
| Total | 100.0 |

### [Example 17] (1st component: liquid, 2nd component: liquid)

| [First component] | Formulation |
|---|---|
| | (mass%) |
| Butyrolactone thiol | 2.0 |
| Cysteamine hydrochloride | 0.5 |
| Propylene glycol | 3.0 |
| Polyoxyethylene (20) cetyl ether | 2.0 |
| Monoethanolamine | 2.0 |
| Hydroxy-ether-amine compound | 0.5 |
| (R¹= formula (III)/R⁵=C₂₀H_{41,} R²⁻³=CH₃, n=1, X=Cl) | |
| Hydroxy-ether-amine quaternary ammonium salt | 0.1 |
| (R¹= formula (III)/R⁵=C₁₈H₃₇, R²⁻⁴=CH_{3,} n=1, X=Br) | |
| Sodium dihydrogen phosphate | 0.2 |
| Disodium hydrogen phosphate | 0.8 |
| Perfume | 0.2 |
| Purified water | balance |
| Total | 100.0 |
| | |

| [Second component] | Formulation |
|---|---|
| | (mass%) |
| Sodium bromate | 8.0 |
| Hydroxy-ether-amine quaternary ammonium salt | 3.0 |
| (R¹= formula (III)/R⁵=C₁₈H₃₇, R²⁻⁴=CH_{3,} n=1, X=C₂H₅SO₄) | |
| Potassium dihydrogen phosphate | 0.1 |
| Disodium hydrogen phosphate | 0.2 |
| Palm oil fatty acid methyl tauride sodium | 1.5 |
| solution (30%) | |
| Purified water | balance |
| Total | 100.0 |

### [Example 18] (1st component: liquid, 2nd component: emulsion)

| [First component] | Formulation |
|---|---|
| | (mass%) |
| DL-Cysteine hydrochloride | 6.5 |
| N-Acetyl-L-cysteine | 1.5 |
| Ammonium thioglycolate solution (50%) | 2.0 |
| Monoethanolamine | 3.5 |
| Soybean protein | 0.1 |
| Trisodium edetate | 0.1 |
| Perfume | 0.2 |
| Purified water | balance |
| Total | 100.0 |
| | |

| [Second component] | Formulation |
|---|---|
| | (mass%) |
| Aqueous hydrogen peroxide (30.0%) | 6.7 |
| Hydroxy-ether-amine quaternary ammonium salt | 5.0 |
| (2) (R¹= formula(III)/R⁵=C₁₈H₃₇, R²⁻⁴=CH₃, n=1, X=Cl) | |
| Phosphoric acid | 0.1 |
| Pentasodium diethylenetriaminepentaacetate | 0.2 |
| solution (40%) | |
| Cetanol | 0.5 |
| Myristyl alcohol | 0.5 |
| Stearyltrimethylammonium chloride | 1.0 |
| POE (8) cetyl ether | 0.2 |
| Acrylic acid·alkyl methacrylate copolymer | 0.3 |
| Purified water | balance |
| Total | 100.0 |

### [Example 19] (1st component: emulsion, 2nd component: emulsion)

| [First component] | Formulation |
|---|---|
| | (mass%) |
| DL-Cysteine hydrochloride | 6.5 |
| N-Acetyl-L-cysteine | 1.5 |
| Ammonium thioglycolate solution (50%) | 2.0 |
| Cetanol | 1.0 |
| Monoethanolamine | 3.5 |
| L-Glutamic acid | 0.1 |
| High-polymerized methylpolysiloxane | 1.0 |
| Trisodium edetate | 0.1 |
| Perfume | 0.2 |
| Purified water | balance |
| Total | 100.0 |
| | |

| [Second component] | Formulation |
|---|---|
| | (mass%) |
| Aqueous hydrogen peroxide (35.0%) | 6.7 |
| Hydroxy-ether-amine compound | 2.0 |
| (R¹= formula (III)/R⁵=C₁₄H₂₉, R²⁻³=CH₃, n=1) | |
| Phosphoric acid | 0.1 |
| Pentasodium diethylenetriaminepentaacetate | 0.2 |
| solution (40%) | |
| Cetanol | 0.5 |
| Myristyl alcohol | 0.5 |
| Stearyltrimethylammonium chloride | 1.0 |
| POE (8) cetyl ether | 0.2 |
| Acrylic acid·alkyl methacrylate copolymer | 0.3 |
| Purified water | balance |
| Total | 100.0 |

### [Example 20] (1st component: liquid, 2nd component: cream)

| [First component] | Formulation |
|---|---|
| | (mass%) |
| Sodium pyrosulfite | 3.0 |
| Urea | 3.0 |
| Monoethanolamine | 3.0 |
| 2-Amino-2-methylpropanol | 1.0 |
| Hydroxy-ether-amine compound | 0.1 |
| (R¹= formula (III)/R⁵=C₁₆H₃₃, R²⁻³=CH₃, n=1) | |
| Hydrolyzed silk liquid | 0.1 |
| Disodium edetate | 0.1 |
| Perfume | 0.2 |
| Purified water | balance |
| Total | 100.0 |
| | |

| [Second component] | Formulation |
|---|---|
| | (mass%) |
| Sodium bromate | 1.0 |
| Potassium dihydrogen phosphate | 0.1 |
| POE (3) sodium lauryl sulfate solution (30%) | 0.5 |
| Behenyl alcohol | 0.3 |
| Cetostearyl alcohol | 1.5 |
| Hydroxy-ether-amine compound | 3.0 |
| (R¹= formula(III)/R⁵=C₂₂H₄₅, R²⁻³=CH₃, n=1) | |
| POE (50) oleyl ether | 0.2 |
| Sodium polyacrylate | 0.1 |
| Purified water | balance |
| Total | 100.0 |

### [Example 21] (1st component: cream, 2nd component: cream)

| [First component] | Formulation |
|---|---|
| | (mass%) |
| Ammonium thioglycolate solution (50%) | 4.0 |
| Ammonium thiolactate solution (60%) | 3.0 |
| N,N'-Diacetylcystine | 1.0 |
| Monoethanolamine | 1.3 |
| Behenyl alcohol | 3.0 |
| Cetanol | 3.0 |
| Distearyldimethylammonium chloride (75%) | 1.0 |
| POE (15) oleyl ether | 0.5 |
| Cationized cellulose | 1.0 |
| Hydroxy-ether-amine compound | 1.0 |
| (R¹= formula (III)/R⁵=C₁₀H₂₁, R²⁻³=CH₃, n=1) | |
| Disodium edetate | 0.1 |
| Perfume | 0.2 |
| Purified water | balance |
| Total | 100.0 |
| | |

| [Second component] | Formulation |
|---|---|
| | (mass%) |
| Aqueous hydrogen peroxide (30.0%) | 6.8 |
| Phenacetin | 0.1 |
| Phosphoric acid | 0.2 |
| Disodium hydrogen phosphate | 0.1 |
| Methyl paraben | 0.1 |
| Behenyl alcohol | 3.0 |
| Cetostearyl alcohol | 3.0 |
| N-Myristoylmethyl-N-taurine sodium | 1.0 |
| POE (15) oleyl ether | 0.2 |
| Aminopropyldimethicone | 0.1 |
| Purified water | balance |
| Total | 100.0 |

### [Example 22] (1st component: cream, 2nd component: cream)

| [First component] | Formulation |
|---|---|
| | (mass%) |
| Acetylcysteine | 0.5 |
| Methylphenylpolysiloxane | 2.0 |
| Hydroxy-ether-amine quaternary ammonium salt | 1.0 |
| (R¹= formula (III)/R⁵=C₁₈H₃₇, R²⁻⁴=CH₃, n=1, X=C₂H₅SO₄) | |
| Alkyltrimethylammonium chloride | 1.0 |
| Distearyldimethylammonium chloride | 0.5 |
| Cetanol | 1.0 |
| Behenyl alcohol | 1.0 |
| 2-Methacryloyloxyethylenephosphorylcholine polymer | 5.0 |
| Monoethanolamine | 0.5 |
| propylene glycol | 10.0 |
| Trisodium edetate | 0.1 |
| Perfume | 0.2 |
| Purified water | balance |
| Total | 100.0 |
| | |

| [Second component] | Formulation |
|---|---|
| | (mass%) |
| Aqueous hydrogen peroxide (35.0%) | 16.0 |
| Hydroxy-ether-amine quaternary ammonium salt | 5.0 |
| (R¹= formula (III)/R⁵=C₁₈H₃₇, R²⁻⁴=CH₃, n=1, X=Cl) | |
| Cetyl alcohol | 9.0 |
| Stearyl alcohol | 6.0 |
| Glyceryl monomyristate | 2.0 |
| Polyoxyethylene (40) cetyl ether | 2.0 |
| Dipropylene glycol | 2.0 |
| Purified water | balance |
| Total | 100.0 |

### [Example 23] (1st component: cream, 2nd component: cream)

| [First component] | Formulation |
|---|---|
| | (mass%) |
| Ammonium thioglycolate solution (50%) | 4.0 |
| Ammonium thiolactate solution (60%) | 3.0 |
| N,N'-Diacetylcystine | 1.0 |
| 2-Mercapto-4-butyrolactone | 2.0 |
| Monoethanolamine | 1.3 |
| Behenyl alcohol | 3.0 |
| Cetanol | 3.0 |
| Distearyldimethylammonium chloride (75%) | 1.0 |
| POE (15) oleyl ether | 0.5 |
| POE (30) phytosterol | 1.0 |
| L-Glutamic acid | 1.0 |
| Glycolic acid | 0.5 |
| Lactic acid | 0.5 |
| Pyrrolidonecarboxylic acid | 5.0 |
| Hydroxymethoxybenzophenonesulfonic acid | 5.0 |
| Hydroxypropylchitosan solution | 1.0 |
| Dimer acid amide | 0.5 |
| Octylguanidine sulfate | 15.0 |
| Cationized cellulose | 1.0 |
| Hydroxy-ether-amine quaternary ammonium salt | 2.0 |
| (R¹= formula(III)/R⁵=C₂₂H₂₅, R²⁻⁴=CH₃, n=1, X=Cl) | |
| Gemini-type cationic activator (2) | 1.0 |
| (R^{6,7}=C₁₂H₂₅, R⁸⁻¹¹=CH₃, Y=Cl) | |
| Dimethyldiallylammonium chloride-acrylic acid copolymer solution (40%) | 1.5 |
| Dimethylsiloxane-methyl(aminopropyl)siloxane copolymer | 2.0 |
| Vinylpyrrolidone-N,N- | 0.3 |
| dimethylaminoethylmethacrylic acid copolymer diethylsulfate salt | |
| Amodimethicone (40% emulsion) | 2.0 |
| Hydroxyethyl cellulose dimethyldiallylammonium chloride | 1.0 |
| Cyclic silicone | 1.0 |
| High-polymerized methylpolysiloxane (no. av. polymn. degree: 3,800) | 1.5 |
| Acrylic acid methacrylamide propyltrimethylammonium chloride | 1.0 |
| Methyl acrylate copolymer solution (Merquat 2001) | 1.0 |
| d-Limonene | 0.1 |
| Arginine | 0.2 |
| Proline | 0.2 |
| Glycylglycine | 2.0 |
| High-molecule keratin | 0.3 |
| Hydrogenetaed soybean lecithin | 0.1 |
| Camomilla extract | 0.3 |
| Seaweed extract | 0.5 |
| Aloe extract | 0.5 |
| Pearl protein | 0.5 |
| Vitamin E | 0.5 |
| Hydrolyzed sericine | 0.5 |
| Matricaria extract | 0.2 |
| Jojoba oil | 2.0 |
| Wheat germ oil | 1.0 |
| Honey | 1.0 |
| Disodium edetate | 0.1 |
| Perfume | 0.2 |
| Purified water | balance |
| Total | 100.0 |

| | |
|---|---|
| [Second component] | Formulation (mass%) |
| Aqueous hydrogen peroxide (30.0%) | 6.8 |
| Phenacetin | 0.1 |
| Phosphoric acid | 0.2 |
| Disodium hydrogen phosphate | 0.1 |
| Methyl paraben | 0.1 |
| Lanolin | 2.0 |
| Behenyl alcohol | 3.0 |
| Cetostearyl alcohol | 3.0 |
| Hydroxy-ether-amine compound (1) | 0.5 |
| (R¹= formula (III)/R⁵=C₁₈H₃₇, R²⁻³=CH₃, n=1) | |
| Gemini-type cationic activator (1) | 2.0 |
| (R^{6,7}=C₁₈H₃₇, R⁸⁻¹¹=CH₃, Y=Cl) | |
| N-Myristoylmethyl-N-taurine sodium | 1.0 |
| POE (15) oleyl ether | 0.2 |
| 1,2-Pentanediol | 5.0 |
| Aminopropyldimethicone | 0.1 |
| Purified water | balance |
| Total | 100.0 |

## Claims

1. A hair shape-controlling composition comprising a first component containing a reducing agent and an alkaline agent, and a second component containing an oxidizing agent, **characterized in that** the composition comprises a hydroxy-ether-amine compound represented by formula (I): 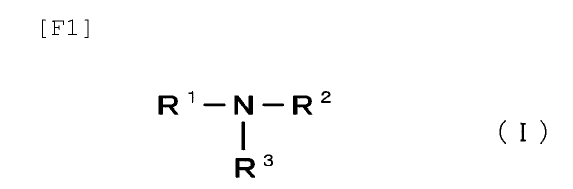 or a quaternary ammonium salt thereof represented by formula (II) : 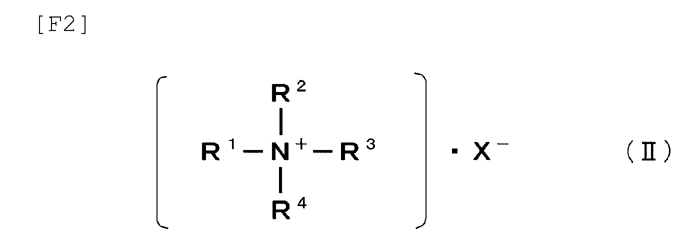 in formulas (I) and (II), each of R¹, R², R³, and R⁴ represents a C1 to C3 alkyl group, a C1 to C3 hydroxyalkyl group, or a group represented by formula (III): 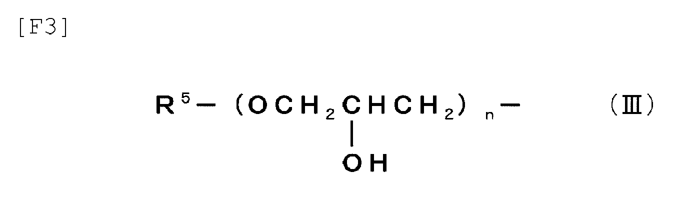 wherein R⁵ represents a linear or branched C6 to C24 alkyl, alkenyl, or hydroxyalkyl group; and n is an integer from 1 to 5; at least one of R¹ to R³ is a group represented by formula (III); and X represents a halogen atom or a C1 or C2 organic sulfur acid group.

2. A hair shape-controlling composition according to claim 1, which is a permanent wave composition.

3. A hair shape-controlling composition according to claim 1, which is a hair straightening composition.

4. A hair shape-controlling composition according to any one of claims 1 to 3, wherein the reducing agent is thiolactic acid or a salt thereof.
